# EUROPEAN PATENT SPECIFICATION

(11) **EP 1 450 813 B1**
(45) Date of publication and mention of the grant of the patent: **29.08.2007**
(21) Application number: 02772562.1
(22) Date of filing: 28.10.2002
(51) Int. Cl.: A61K 31/5375, A61K 31/4196, A61K 38/08, A61K 31/557, A61K 9/00

(54) **ANTAGONISTS OF PROSTAGLANDIN RECEPTORS EP2 AND/OR EP4 FOR THE TREATMENT OF MENORRHAGIA**
ANTAGONISTEN DER PROSTAGLANDIN EP2 UND/ODER EP4 REZEPTOREN ZUR BEHANDLUNG VON MENORRHAGIE
ANTAGONISTES DE RECEPTEURS DE PROSTAGLANDINE EP2 ET/OU EP4 POUR LE TRAITEMENT DE LA MENORRAGIE

(30) Priority: 31.10.2001 GB 0126094; 09.08.2002 US 402864 P
(43) Date of publication of application: 01.09.2004
(73) Proprietor: MEDICAL RESEARCH COUNCIL, London W1B 1AL (GB)
(72) Inventor: JABBOUR, Henry, N., MRC Human Reproductive Sciences Unit, Edinburgh EH16 4TJ (GB); CRITCHLEY, Hilary, O., D., Center for Reproductive Biology The University of Edinburgh Med. School, Edinburgh EH16 4SB (GB)
(74) Representative: Miles, John Stephen
(86) International application number: PCT/GB2002/004845
(87) International publication number: WO 2003/037348

(56) References cited:
- EP-A- 1 097 922
- WO-A-00/18405
- WO-A-01/10426
- WO-A-01/42281
- WO-A-01/72302
- GB-A- 2 330 307
- US-A- 5 912 006
- US-B1- 6 197 327
- US-B1- 6 211 221
- MACHWATE M ET AL: "Prostaglandin receptor EP4 mediates the bone anabolic effects of PGE2." MOLECULAR PHARMACOLOGY, vol. 60, no. 1, July 2001 (2001-07), pages 36-41, XP002229205 ISSN: 0026-895X cited in the application
- LI XIAODONG ET AL: "Knockout of the murine prostaglandin EP2 receptor impairs osteoclastogenesis in vitro." ENDOCRINOLOGY, vol. 141, no. 6, June 2000 (2000-06), pages 2054-2061, XP002229451 ISSN: 0013-7227
- HILLOCK C J ET AL: "Inhibitory prostanoid EP receptors in human non-pregnant myometrium." EUROPEAN JOURNAL OF PHARMACOLOGY. NETHERLANDS 28 JUL 1999, vol. 378, no. 1, 28 July 1999 (1999-07-28), pages 99-108, XP002229207 ISSN: 0014-2999 cited in the application
- FRASER I S ET AL: "LONG-TERM TREATMENT OF MENORRHAGIA WITH MEFENAMIC-ACID" OBSTETRICS AND GYNECOLOGY, vol. 61, no. 1, 1983, pages 109-112, XP009004913 ISSN: 0029-7844
- DUNN C J ET AL: "TRANEXAMIC ACID A REVIEW OF ITS USE IN SURGERY AND OTHER INDICATIONS" DRUGS, ADIS INTERNATIONAL LTD, AT, vol. 57, no. 6, June 1999 (1999-06), pages 1005-1032, XP008005661 ISSN: 0012-6667
- STIRRAT GORDON M: "Choice of treatment for menorrhagia." LANCET (NORTH AMERICAN EDITION), vol. 353, no. 9171, 26 June 1999 (1999-06-26), pages 2175-2176, XP002229208 ISSN: 0099-5355 cited in the application

## Description

The present invention relates to the treatment of menorrhagia.

Menorrhagia is over-abundance of the menstrual discharge.

Menorrhagia affect many women, particularly in the Western world, and represent a significant health problem. At least one in 20 women in the UK aged between 34 and 49 years will consult their general practitioners because of menstrual problems. These women account for more than one in ten, of all gynaecological referrals and cost the NHS in excess of £7 million per year for medical prescriptions alone. Perceived abnormal vaginal bleeding is said to account for 70% of the at least 70 000 hysterectomies done each year.

At present, the treatments used for menorrhagia include tranexamic acid or mefenamic acid. In severe cases the treatment is hysterectomy (vaginal or abdominal) but this is a major operation with serious morbidity and some risk of death. A review of treatments for menorrhagia is Stirrat (1999) The Lancet 353, 2175-2176. The development of further and alternative therapies is desirable.

The inventors now propose that an alternative method for treating menorrhagia is to use antagonists of the prostaglandin EP2 and/or EP4 receptor. This approach is believed more likely to be effective in more women than other drug treatments. The EP2 and/or EP4 receptor antagonists are deliverable *in utero.*

Prostaglandin E₂ elicits its autocrine/paracrine effects on target cells through interaction with transmembrane G protein coupled receptors. To date four main sub-types of PGE₂ receptors have been identified based on responses to agonists and antagonists and are pharmacologically divided into EP1, EP2, EP3 and EP4 which utilise alternate and in some cases opposing intracellular signalling pathways. EP2 and EP4 increase cAMP levels *via* G_{αs}.

The EP2 and EP4 receptors are known to be expressed in human nonpregnant endometrium. No differences in EP2 receptor mRNA expression were detected in tissue collected across the menstrual cycle; however, EP4 receptor mRNA expression was significantly higher in the late proliferative stage than in early, mid and late secretory stage endometrium (Milne et al (2001) J. Clin. Endocrinol. Metab. 86, 4453-4459. The inventors now show that EP2 and EP4 receptors are overexpressed in women with menorrhagia and in women with menorrhagia it should prove beneficial to treat with receptor antagonists in order to block the signalling pathway and ultimately transcription of target genes that may mediate vascular function/dysfunction and excessive bleeding.

WO 01/72302 relates to EP4 receptor antagonists and their use for the treatment of headache.

WO 01/10426 relates to EP4 receptor antagonists and their use for the treatment of neuropathic pain and colon cancer.

GB 2 330 307 relates to EP4 receptor antagonists and their use for the treatment of bone diseases.

US Patent No 6,197,327 B1 relates to an intravaginal device and method for treatment of dysmenorrhoea.

A fist aspect of the invention provides the use of an antagonist of a prostaglandin EP2 and/or EP4 receptor in the manufacture of a medicament for treating menorrhagia.

It is possible to have menorrhagia and dysmenorrhoea together and the medicament may be used to treat both conditions in the same patient.

The patient may be any patient who is suffering from menorrhagia or a patient who is at risk from this condition. Any premenopausal or perimenopausal woman is at risk of menorrhagia; however, menorrhagia is more common at the beginning and end of a woman's reproductive life so typically there is a greater risk when a woman's periods first start and in women over 40 years of age. The patient to be treated may be any female individual who would benefit from such treatment. Typically and preferably the patient to be treated is a human female. However, the methods of the invention may be used to treat female mammals, such as the females of the following species: cows, horses, pigs, sheep, cats and dogs. Thus, the methods have uses in both human and veterinary medicine.

The prostaglandin EP2 receptor antagonist may be any suitable EP2 receptor antagonist. Similarly, the prostaglandin EP4 receptor antagonist may be any suitable EP4 receptor antagonist By "suitable" we mean that the antagonist is one which may be administered to the patient. The receptor antagonists are molecules which bind to their respective receptors, compete with the natural ligand (PGE₂) and inhibit the initiation of the specific receptor-mediated signal transduction pathways. The receptor antagonists are typically selective to the particular receptor and typically have a higher binding affinity to the receptor than the natural ligand. Although antagonists with a higher affinity for the receptor than the natural ligand are preferred, antagonists with a lower affinity may also be used, but it may be necessary to use these at higher concentrations. Preferably, the antagonists bind reversibly to their cognate receptor. Typically, antagonists are selective for a particular receptor and do not affect the other receptor; thus, typically, an EP2 receptor antagonist binds the EP2 receptor but does not substantially bind the EP4 receptor, whereas an EP4 receptor antagonist binds the EP4 receptor but does not substantially bind the EP2 receptor. Preferably, the EP2 or EP4 receptor antagonist is selective for the particular receptor subtype. By this is meant that the antagonist has a binding affinity for the particular receptor subtype which is at least ten-fold higher than for at least one of the other EP receptor subtypes. Thus, selective EP4 receptor antagonists have at least a ten-fold higher affinity for the EP4 receptor than any of the EP1, EP2 or EP3 receptor subtypes.

It is particularly preferred that the EP2 or EP4 receptor antagonist is selective for its cognate receptor.

The EP2 or EP4 receptor antagonists are typically for administration in an effective amount to combat the menorrhagia. Thus, the medicaments containing the antagonist may be used to alleviate symptoms (ie are used palliatively) or may be used to treat the condition. The antagonist may be for administration prophylactically (and by "treating" we include prophylactic treatment). The antagonist may be for administration by any suitable route, and in any suitable form. It is desirable to administer an amount of the EP2 or EP4 receptor antagonist that is effective in preventing or alleviating or ameliorating or curing the menorrhagia.

EP2 receptor antagonists include AH6809 (Pelletier et al (2001) Br. J. Pharmacol. 132, 999-1008).

EP4 receptor antagonists include AH23848B (developed by Glaxo) and AH22921X (Pelletier et al (2001) Br. J. Pharmacol. 132, 999-1008. The chemical name for AH23848B is ([1alpha(z), 2beta5alpha]-(+/-)-7-[5-[[(1,1'-biphenyl)-4-yl]methoxy]-2-(4-morpholinyl)-3-oxo-cyclopentyl]-4-heptenoic acid) (see Hillock & Crankshaw (1999) Eur. J. Pharmacol. 28, 99-108). EP4RA (Li i (2000) Endocrinology 141, 2054-61) is an EP(4) - selective ligand (Machwate et al (2001) Mol. Pharmacol. 60: 36-41). The omega-substituted prostaglandin E derivatives described in WO 00/15608 (EP 1 114 816) (Ono Pharm Co Ltd) bind EP4 receptors selectively and may be EP4 receptor antagonists.

Peptides described in WO 01/42281 (Hopital Sainte-Justine) eg: IFTSYLECL, IFASYECL, IFTSAECL, IFTSYEAL, ILASYECL, IFTSTDCL, TSYEAL (with 4-biphenyl alanine), TSYEAL (with homophenyl alanine) are also described as EP4 receptor antagonists, as are some of the compounds described in WO 00/18744 (Fujisawa Pharm Co Ltd). The 5-thia-prostaglandin E derivatives described in WO 00/03980 (EP 1 097 922) (Ono Pharm Co Ltd) may be EP4 receptor antagonists.

EP4 receptor antagonists are also described in WO 01/10426 (Glaxo), WO 00/21532 (Merck) and GB 2 330 307 (Glaxo).

WO 00/21532 describes the following as EP4 receptor antagonists:
5-butyl-2,4-dihydro-4-[[2'-[N-(3-chloro-2-thiophenecarbonyl)sulfamoyl]biphenyl-4-yl]methyl]-2-{2-(trifluoromethyl)phenyl]-1,2,4-triazol-3-one potassium salt;
5-butyl-2,4-dihydro-4-[[2'-[N-(2-methyl-3-furoyl)sulfamoyl]biphenyl-4-yl]methyl]-2-{2-(trifluoromethyl)phenyl]-1,2,4-triazol-3-one;
5-butyl-2,4-dihydro-4-[[2'-[N-(3-methyl-2-thiophenecarbonyl)sulfamoyl]biphenyl-4-yl]methyl]-2-{2-(trifluoromethyl)phenyl]-1,2,4-tiazol-3-one;
5-butyl-2,4-dihydro-4-[[2'-[N-(2-thiophenecarbonyl)sulfamoyl]biphenyl-4-yl]methyl]-2-{2-(trifluoromethyl)phenyl]-1,2,4-triazol-3-one;
5-butyl-2,4-dihydro-4-[[2'-[N-[2-(methypyrrole)carbonyl]sulfamoyl]biphenyl-4-yl]methyl]-2-{2-(trifluoromethyl)phenyl]-1,2,4-triazol-3-one.

GB 2 330 307 describes [1α(Z), 2β,5α]-(±)-7-[5-[[(1,1'-biphenyl)-4-yl]methoxy]-2-(4-morpholinyl)-3-oxocyclopentyl]-4-heptenoic acid and [1R[1α(z),2β,5α]]-(-)-7-[5-[[(1,1,'-biphenyl)-4-yl]methoxy]-2-(4-morpholinyl)-3-oxocyclopentyl]-4-heptenoic acid.

WO 00/18405 (Pharmagene) describes the EP4 receptor antagonists AH22921 and AH23848 (which are also described in GB 2 028 805 and US4, 342, 756). WO 01/72302 (Pharmagene) describes further EP4 receptor antagonists, particularly those in the general formula (I) shown on page 8 *et seq.*

It will be appreciated that one or more EP2 receptor antagonists, or one or more EP4 receptor antagonists, may be administered to the patient. It will also be appreciated that a combination of one or more EP2 or EP4 receptor antagonists may be administered to the patient Preferably, an EP4 receptor antagonist is administered to the patient.

The aforementioned EP2 or EP4 receptor antagonists, or a formulation thereof, may be for administration by any conventional method including oral and parenteral (eg subcutaneous or intramuscular) injection. The treatment may consist of a single dose or a plurality of doses over a period of time.

While it is possible for a compound of the invention to be for administration alone, it is preferable to present it as a pharmaceutical formulation, together with one or more acceptable carriers. The carrier(s) must be "acceptable" in the sense of being compatible with the compound of the invention and not deleterious to the recipients thereof. Typically, the carriers will be water or saline which will be sterile and pyrogen free.

The formulations may conveniently be presented in unit dosage form and may be prepared by any of the methods well known in the art of pharmacy. Such methods include the step of bringing into association the antagonist with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations in accordance with the present invention suitable for oral administration may be presented as discrete units such as capsules, cachets or tablets, each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (eg povidone, gelatin, hydroxypropylmethyl cellulose), lubricant, inert diluent, preservative, disintegrant (eg sodium starch glycolate, cross-linked povidone, cross-linked sodium carboxymethyl cellulose), surface-active or dispersing agent Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethylcellulose in varying proportions to provide desired release profile.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier. Buccal administration is also preferred.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

Certain EP2 and EP4 receptor antagonists are proteins or peptides. Proteins and peptides may be delivered using an injectable sustained-release drug delivery system. These are designed specifically to reduce the frequency of injections. An example of such a system is Nutropin Depot which encapsulates recombinant human growth hormone (rhGH) in biodegradable microspheres that, once injected, release rhGH slowly over a sustained period.

The protein and peptide can be administered by a surgically implanted device that releases the drug directly to the required site. For example, Vitrasert releases ganciclovir directly into the eye to treat CMV retinitis. The direct application of this toxic agent to the site of disease achieves effective therapy without the drug's significant systemic side-effects.

Electroporation therapy (EPT) systems can also be employed for the administration of proteins and peptides. A device which delivers a pulsed electric field to cells increases the permeability of the cell membranes to the drug, resulting in a significant enhancement of intracellular drug delivery.

Proteins and peptides can be delivered by electroincorporation (EI). EI occurs when small particles of up to 30 microns in diameter on the surface of the skin experience electrical pulses identical or similar to those used in electroporation. In EI, these particles are driven through the stratum corneum and into deeper layers of the skin. The particles can be loaded or coated with drugs or genes or can simply act as "bullets" that generate pores in the skin through which the drugs can enter.

An alternative method of protein and peptide delivery is the ReGel injectable system that is thermo-sensitive. Below body temperature, ReGel is an injectable liquid while at body temperature it immediately forms a gel reservoir that slowly erodes and dissolves into known, safe, biodegradable polymers. The EP2 or EP4 receptor antagonist is delivered over time as the biopolymers dissolve.

Protein and peptide pharmaceuticals can also be delivered orally. The process employs a natural process for oral uptake of vitamin B₁₂ in the body to co-deliver proteins and peptides. By riding the vitamin B₁₂ uptake system, the protein or peptide can move through the intestinal wall. Complexes are synthesised between vitamin B₁₂ analogues and the drug that retain both significant affinity for intrinsic factor (IF) in the vitamin B₁₂ portion of the complex and significant bioactivity of the drug portion of the complex.

Proteins and polypeptides can be introduced to cells by "Trojan peptides". These are a class of polypeptides called penetratins which have translocating properties and are capable of carrying hydrophilic compounds across the plasma membrane. This system allows direct targetting of oligopeptides to the cytoplasm and nucleus, and may be non-cell type specific and highly efficient. See Derossi et al (1998), Trends Cell Biol 8, 84-87.

The antagonist is for administration at a dose (or in multiple doses) which produces a beneficial therapeutic effect in the patient. Suitable doses may be determined by the physician. The dose to be administered is determined upon age, body weight, mode of administration, duration of the treatment, and pharmacokinetic and toxicological properties of the antagonist.

It is preferred if the antagonist is for administration orally. It is further preferred if the antagonist is for administration to the female reproductive system. For example, the antagonist may suitably be for administration intravaginally using, for example, a gel or cream or vaginal ring or tampon. The antagonist may also advantageously be for administration using an intrauterine device.

Typically, the gel or cream is one which is formulated for administration to the vagina. It may be oil based or water based. Typically, the antagonist is present in the cream or gel in a sufficient concentration so that an effective amount is for administration in a single (or in repeated) application.

Typically, the vaginal ring comprises a polymer which formed into a "doughnut" shape which fits within the vagina. The antagonist is present within the polymer, typically as a core, which may dissipate through the polymer and into the vagina and/or cervix in a controlled fashion. Vaginal rings are known in the art. The vaginal ring may be disposable and is retained intravaginally during the woman's period and therefore contains sufficient antagonist to be released and to be effective during the woman's period. Alternatively, the vaginal ring may be used over a time interval of around three months to one year, during which time sufficient antagonist is released to have a beneficial effect over that period of time. It will be appreciated that the polymer from which the ring is made, the size and shaper of the ring and the content of antagonist, as well as other parameters, may be selected by reference to whether the ring is for use in one cycle or for longer spells.

Typically, the tampon is impregnated with the antagonist and that a sufficient amount of the antagonist is present in the tampon bearing in mind that more than one tampon is generally used in a single day, for example up to 10 to 15 tampons in a single day.

Typically, the intrauterine device is for placing in the uterus over extended periods of time, such as between one and five years. Typically, the intrauterine device comprises a plastic frame, often in the shape of a "T" and contains sufficient antagonist to be released over the period of use. The antagonist is generally present within or encompassed by a slow-release polymer which forms part of the device, such as in the form of a "sausage" of antagonist which wraps around the long arm of the "T" which is typically covered with a controlled-release membrane. Intrauterine devices are known in the art.

The invention also provides combinations (such as in a pharmaceutical formulation) of one or more EP2 and/or EP4 receptor antagonists and one or more agents presently used to treat menorrhagia, such as tranexamic acid or mefenamic acid.

The invention will now be described in more detail with reference to the following non-limiting Examples and Figure.

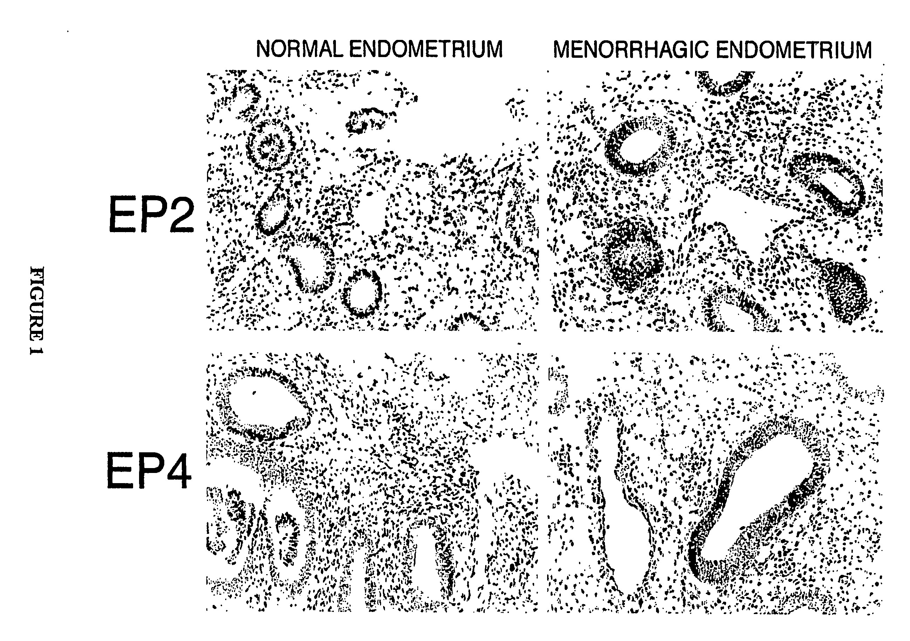

Figure 1 shows endometrial sections from menorrhagic and control women stained with antibodies to the EP2 receptor and EP4 receptor as described in Example 2.

### Example 1: Expression of EP2 and EP4 receptors in uterine tissue of women with menorrhagia compared to women with no menorrhagia

Uterine tissue is collected by biopsy from women with known indication of menorrhagia and/or dysmenorrhoea and women who have normal uterine function. The tissue is assessed for the expression of EP receptors including EP2 and EP4. This is assessed using various molecular techniques. The signalling of these receptors in response to PGE₂ is assessed. Tissue is cultured for various time in the presence or absence of PGE₂ and the second messenger cAMP is measured in response to these treatments.

Expression of EP2 and/or EP4 receptors is elevated in the uterine tissue that comes from women with a known history with menorrhagia and/or dysmenorrhoea. Moreover, the signalling events in response to PGE₂ is augmented in these patients.

Hence in women with these conditions, it should prove beneficial to treat with receptor antagonists in order to block the signalling pathway and ultimately transcription of target genes that may mediate vascular function/dysfunction and excessive bleeding.

### Example 2: Elevated expression of EP2 and EP4 receptors in endometrium of menorrhagic women compared to control women

### Methods

Endometrial sections (5 µm) collected from two women classed as control (with <80 ml blood loss per cycle) or menorrhagic (with >80 ml blood loss per cycle) were dewaxed in xylene and rehydrated using decreasing grades of ethanol. After rinsing in PBS, endogenous peroxidase activity was quenched with 3% H₂O₂ in methanol. Non-immune swine serum (10% serum in PBS) was applied for 20 min before overnight incubation at 4°C with primary antibody. An avidin-biotin peroxidase detection system was then applied (DAKO Ltd, UK) with 3,3'-diaminobenzidine (DAB) as the chromagen. Sections were counter stained with Harris's haematoxylin before mounting. The primary antibodies used in this study were raised in rabbits against human EP2 or EP4 receptor peptide sequences (Cayman Chemicals, USA). The antibody was used at a 1:250 dilution. All treatments were carried out at room temperature unless otherwise specified.

### Results

Staining for both EP2 and EP4 receptors was localised in the glandular epithelial cells and endothelial cells. Lower intensity of staining was observed in the endometrial samples collected from the woman with normal bleeding pattern as compared with endometrium collected with women suffering from menorrhagia. This indicates a higher expression pattern of the two receptors in the latter group of women.

The results are shown in Figure 1.

### Example 3: Treatment of menorrhagia with an EP2 receptor antagonist

A woman presents to her physician with symptoms of menorrhagia. The physician diagnoses menorrhagia. The woman is administered an effective dose of AH6809.

### Example 5: Suppository

| | mg/suppository |
|---|---|
| AH22921 (63 µm)* | 250 |
| Hard Fat, BP (Witepsol H15- Dynamit Nobel) | 1770 |
| | 2020 |

| | |
|---|---|
| *The antagonist AH22921 is used as a powder wherein at least 90% of the particles are of 63 µm diameter or less. | |

One fifth of the Witepsol H15 is melted in a steam-jacketed pan at 45°C maximum. The active ingredient is sifted through a 200 µm sieve and added to the molten base with mixing, using a silverson fitted with a cutting head, until a smooth dispersion is achieved. Maintaining the mixture at 45°C, the remaining Witepsol H15 is added to the suspension and stirred to ensure a homogenous mix. The entire suspension is passed through a 250 µm stainless steel screen and, with continuous stirring, is allowed to cool to 40°C. At a temperature of 38°C to 40°C 2.02 g of the mixture is filled into suitable plastic moulds. The suppositories are allowed to cool to room temperature.

### Example 6: Pessaries

| | mg/pessary |
|---|---|
| AH23848B | 250 |
| Anhydrate Dextrose | 380 |
| Potato Starch | 363 |
| Magnesium Stearate | 7 |
| | 1000 |

The above ingredients are mixed directly and pessaries prepared by direct compression of the resulting mixture.

### Example 7: Vaginal ring

A vaginal ring containing 5-butyl-2,4-dihydro-4-[[2'-[N-(3-chloro-2-thiophenecarbonyl)sulfamoyl]biphenyl-4-yl]methyl]-2-{2-(trifluoromethyl)phenyl]-1,2,4-triazol-3-one potassium salt; is produced using core extrusion technology.

### Example 8: Intrauterine device

An intrauterine device containing AH6809 is produced using standard technology.

### Example 9: Tampon

A tampon for treating menorrhagia and/or dysmenorrhoea is produced by impregnating a standard tampon with an effective dose of 5-butyl-2,4-dihydro-4-[[2'-[N-[2-(methypyrrole)carbonyl]sulfamoyl]biphenyl-4-yl]methyl]-2-{2-(trifluoromethyl)phenyl]-1,2,4-triazol-3-one.

## Claims

1. Use of an antagonist of a prostaglandin EP2 and/or EP4 receptor in the manufacture of a medicament for treating menorrhagia in a patient.

2. The use according to Claim 1 wherein the antagonist is an antagonist of a prostaglandin EP2 receptor.

3. The use according to Claim 1 wherein the antagonist is an antagonist of a prostaglandin EP4 receptor.

4. The use according to any one of Claims 1 to 3 wherein the patient premenopausal or perimenopausal.

5. The use according to Claim 1 wherein the medicament contains any one or more of AH6809, AH23848B, AH22921X, IFTSYLECL, IFASYECL, IFTSAECL, IFTSYEAL, ILASYECL, TFTSTDCL, TSYEAL (with 4-biphenylalanine), TSYEAL (with homophenylalanine), 5-butyl-2,4-dihydro-4-[[2'-[N-(3-chloro-2-thiophenecarbonyl)sulfamoyl]biphenyl-4-yl]methyl]-2-{2-(trifluoromethyl)phenyl]-1,2,4-triazol-3-one potassium salt, 5-butyl-2,4-dihydro-4-[[2'-[N-(2-methyl-3-furoyl)sulfamoyl]biphenyl-4-yl]methyl]-2-{2-(trifluoromethyl)phenyl]-1,2,4-triazol-3-one, 5-butyl-2,4-dihydro-4-[[2'-[N-(3-methyl-2-thiophenecarbonyl)sulfamoyl]biphenyl-4-yl]methyl]-2-{2-(trifluoromethyl)phenyl]-1,2,4-triazol-3-one, 5-butyl-2,4-dihydro-4-[[2'-[N-(2-thiophenecarbonyl)sulfamoyl]biphenyl-4-yl]methyl]-2-{2-(trifluoromethyl)phenyl]-1,2,4-triazol-3-one, and 5-butyl-2,4-dihydro-4-[[2'-[N-[2-(methypyrrole)carbonyl]sulfamoyl]biphenyl-4-yl]methyl]-2-{2-(trifluoromethyl)phenyl]-1,2,4-triazol-3-one.

6. The use according to Claim 2 wherein the EP2 receptor antagonist is any one or more of AH6809.

7. The use according to Claim 3 wherein the EP4 receptor antagonist is any one or more of AH23848B, AH22921X, IFTSYLECL, IFASYECL, IFTSAECL, IFTSYEAL, ILASYECL, IFTSTDCL, TSYEAL (with 4-biphenylalanine), TSYEAL (with homophenylalanine), 5-butyl-2,4-dibydro-4-[[2'-[N-(3-chloro-2-thiophenecarbonyl)sulfamoyl]biphenyl-4-yl]methyl]-2-{2-(trifluoromethyl)phenyl]-1,2,4-triazol-3-one potassium salt, 5-butyl-2,4-dihydro-4-[[2'-[N-(2-methyl-3-furoyl)sulfamoyl]biphenyl-4-yl]methyl]-2-{2-(trifluoromethyl)phenyl]-1,2,4-triazol-3-one, 5-butyl-2,4-dihydro-4-[[2'-[N-(3-methyl-2-thiophenecarbonyl)sulfamoyl]biphenyl-4-yl]methyl]-2-{2-(trifluoromethyl)phenyl]-1,2,4-triazol-3-one, 5-butyl-2,4-dihydro-4-[[2'-[N-(2-thiophenecarbonyl)sulfamoyl]biphenyl-4-yl]methyl]-2-{2-(trifluoromethyl)phenyl]-1,2,4-triazol-3-one, and 5-butyl-2,4-dihydro-4-[[2'-[N-[2-(methypyrrole)carbonyl]sulfamoyl]biphenyl-4-yl]methyl]-2-{2-(trifluoromethyl)phenyl]-1,2,4-triazol-3-one.

8. A vaginal ring or a tampon or an intrauterine device comprising and EP2 and/or an IEP4 receptor antagonist.

9. A combination of any one or more of an EP2 and/or EP4 receptor antagonist and tranexamic acid or mefenamic acid.

10. A Pharmaceutical composition comprising a combination according to Claim 9 and a pharmaceutically acceptable carrier.

## Patentansprüche

1. Verwendung eines Antagonisten eines Prostaglandin-EP2- und/oder -EP4-Rezeptors bei der Herstellung eines Medikaments zur Behandlung von Menstruationsbeschwerden bei einem Patienten.

2. Verwendung nach Anspruch 1, wobei der Antagonist ein Antagonist eines Prostaglandin-EP2-Rezeptors ist.

3. Verwendung nach Anspruch 1, wobei der Antagonist ein Antagonist eines Prostaglandin-EP4-Rezeptors ist.

4. Verwendung nach einem der Ansprüche 1 bis 2, wobei der Patient sich vor dem oder im Klimakterium befindet.

5. Verwendung nach Anspruch 1, wobei das Medikament eines oder mehrere enthält von AH6809, AH23848B, AH22921X, IFTSYLECL, IFASYECL, IFTSAECL, IFTSYEAL, ILASYECL; IFTSTDCL, TSYEAL (mit 4-Biphenylalanin), TSYEAL (mit Homophenylalanin), 5-Butyl-2,4-dihydro-4-[[2'-[N-(3-chlor-2-thiophencarbonyl)sulfamoyl]buphenyl-4-yl]methyl]-2-{2-(trifluormethyl)phenyl]-1,2,4-triazol-3-on-kaliumsalz, 5-Butyl-2,4-dihydro-4-[[2'-[N-(2-methyl-3-furoyl)sulfamoyl]biphenyl-4-yl]methyl]-2-{-(trifluormethyl)phenyl]-1,2,4-triazol-3-on, 5-Butyl-2,4-dihydro-4-[[2'-[N-(3-methyl-2-thiophencarbonyl)sulfamoyl]-biphenyl-4-yl]methyl]-2-{2-(trifluormethyl)phenyl]-1,2,4-triazol-3-on, 5-Butyl-2,4-dihydro-4-[['2-[2-thiophencarbonyl)sulfamoyl]biphenyl-4-yl]methyl]-2-{2-[trifluormethyl]phenyl]-1,2,4-triazol-3-on und 5-Butyl-2,4-dihydro-4-[[2'-[N-[2-(methylpyrrol)carbonyl]sulfamoyl]biphenyl-4-yl]methyl]-2-{2-(trifluormethyl)-phenyl]-1,2,4-triazol-3-on.

6. Verwendung nach Anspruch 2, wobei der EP2-Rezeptor Antagonist einer oder mehrere von AH6809 ist.

7. Verwendung nach Anspruch 3, wobei der EP4-Rezeptor Antagonist einer oder mehrere von AH23848B, AH22921X, IFTSYLECL, IFASYECL, IFTSAECL, IFTSYEAL, ILASYECL; IFTSTDCL, TSYEAL (mit 4-Biphenylalanin), TSYEAL (mit Homophenylalanin), 5-Butyl-2,4-dihydro-4-[[2'-[N-(3-chlor-2-thiophencarbonyl)sulfamoyl]buphenyl-4-yl]methyl]-2-{2-(trifluormethyl)phenyl]-1,2,4-triazol-3-on-kaliumsalz, 5-Butyl-2,4-dihydro-4-[[2'-[N-(2-methyl-3-furoyl)sulfamoyl]biphenyl-4-yl]methyl]-2-{(trifluormethyl)phenyl]-1,2,4-triazol-3-on, 5-Butyl-2,4-dihydro-4-[[2'-[N-(3-methyl-2-thiophencarbonyl)-sulfamoyl]biphenyl-4-yl]methyl]-2-{2-(trifluormethyl)phenyl]-1,2,4-triazol-3-on, 5-Butyl-2,4-dihydro-4-[['2-[2-thiophencarbonyl)sulfamoyl]biphenyl-4-yl]methyl]-2-{2-[trifluoromethyl]phenyl}-1,2,4-triazol 3 on und 5-Butyl-2,4-dihydro-4-[[2'-[N-[2-(methylpyrrol)carbonyl]sulfamoyl]biphenyl-4-yl]methyl]-2-{2-(trifluormethyl)pheny]-1,2,4-triazol-3-on ist.

8. Vaginalring oder Tampon oder intrauterine Einrichtung, die einen EP2- und/oder einen EP4-Rezeptorantagonisten aufweist.

9. Kombination eines oder mehrerer EP2- und/oder EP4-Rezeptorantagonisten mit Tranexamsäure oder Mefenamsäure.

10. Pharmazeutische Zusammensetzung, die eine Kombination nach Anspruch 9 und eine pharmazeutisch akzeptablen Träger aufweist.

## Revendications

1. Utilisation d'un antagoniste d'un récepteur EP2 et/ou EP4 de prostaglandine pour la fabrication d'un médicament pour traiter une hémorragie chez un patient.

2. Utilisation selon la revendication 1, dans laquelle l'antagoniste est un antagoniste d'un récepteur EP2 de prostaglandine.

3. Utilisation selon la revendication 1, dans laquelle l'antagoniste est un antagoniste d'un récepteur EP4 de prostaglandine.

4. Utilisation selon l'une quelconque des revendications 1 à 3, dans laquelle la patiente est préménopausique ou périménopausique.

5. Utilisation selon la revendication 1, dans laquelle le médicament contient l'un quelconque ou plusieurs parmi AH6809, AH23848B, AH22921X, IFTSYLECL, IFASYECL, IFTSAECL, IFTSYEAL, ILASYECL, IFTSTDCL, TSYEAL (avec 4-biphénylalanine), TSYEAL (avec homophénylalanine), sel potassique de 5-butyl-2,4-dihydro-4-[[2'-[N-(3-chloro-2-thiophènecarbonyl)sulfamoyl]biphényl-4-yl]méthyl]-2-{2-(trifluorométhyl)phényl]-1,2,4-triazol-3-one, 5-butyl-2,4-dihydro-4-[[2'-[N-(2-méthyl-3-furoyl)sulfamoyl]biphényl-4-yl]-méthyl]-2-{2-(trifluorométhyl)phényl]-1,2,4-triazol-3-one, 5-butyl-2,4-dihydro-4-[[2'-[N-(3-méthyl-2-thiophènecarbonyl)sulfamoyl]biphényl-4-yl]méthyl]-2-{2-(trifluorométhyl)phényl]-1,2,4-triazol-3-one, 5-butyl-2,4-dihydro-4-[[2'-[N-(2-thiophènecarbonyl)sulfamoyl]biphényl-4-yl]méthyl]-2-{2-(trifluorométhyl)phényl]-1,2,4-triazol-3-one et 5-butyl-2,4-dihydro-4-[[2'-[N-[2-(méthypyrrole)carbonyl]sulfamoyl]biphényl-4-yl]méthyl]-2-{2-(trifluorométhyl)phényl]-1,2,4-triazol-3-one.

6. Utilisation selon la revendication 2, dans laquelle l'antagoniste du récepteur EP2 est l'un quelconque ou plusieurs parmi AH6809.

7. Utilisation selon la revendication 3, dans laquelle l'antagoniste du récepteur EP4 est l'un quelconque ou plusieurs parmi AH23848B, AH22921X, IFTSYLECL, IFASYECL, IFTSAECL, IFTSYEAL, ILASYECL, IFTSTDCL, TSYEAL (avec 4-biphénylalanine), TSYEAL (avec homophénylalanine), sel potassique de 5-butyl-2,4-dihydro-4-[[2'-[N-(3-chloro-2-thiophènecarbonyl)sulfamoyl]biphényl-4-yl]méthyl]-2-{2-(trifluorométhyl)phényl]-1,2,4-triazol-3-one, 5-butyl-2,4-dihydro-4-[[2'-[N-(2-méthyl-3-furoyl)sulfamoyl]biphényl-4-yl]méthyl]-2-{2-(trifluorométhyl)phényl]-1,2,4-triazol-3-one, 5-butyl-2,4-dihydro-4-[[2'-[N-(3-méthyl-2-thiophènecarbonyl)sulfamoyl]biphényl-4-yl]méthyl]-2-{2-(trifluorométhyl)phényl]-1,2,4-triazol-3-one, 5-butyl-2,4-dihydro-4-[[2'-[N-(2-thiophènecarbonyl)sulfamoyl]biphényl-4-yl]méthyl]-2-{2-(trifluorométhyl)phényl]-1,2,4-triazol-3-one et 5-butyl-2,4-dihydro-4-[[2'-[N-[2-(méthypyrrole)carbonyl]sulfamoyl]biphényl-4-yl]méthyl]-2-{2-(trifluorométhyl)phényl]-1,2,4-triazol-3-one.

8. Anneau vaginal ou tampon ou dispositif intra-utérin comprenant un antagoniste des récepteurs EP2 et/ou EP4.

9. Combinaison de l'un quelconque ou de plusieurs d'un antagoniste des récepteurs EP2 et/ou EP4 et d'acide tranexamique ou d'acide méfénamique.

10. Composition pharmaceutique comprenant une combinaison selon la revendication 9 et un véhicule pharmaceutiquement acceptable.
